Europäisches Patentamt

European Patent Office

Office européen des brevets

(19)

(11) Numéro de publication : **0 010 483**
**B1**

(12) # FASCICULE DE BREVET EUROPÉEN

(45) Date de publication du fascicule du brevet :
05.10.83

(51) Int. Cl.³ : **A 61 K 7/42**

(21) Numéro de dépôt : **79400718.7**

(22) Date de dépôt : **08.10.79**

(54) **Composition utilisable notamment comme produit cosmétique permettant un bronzage de la peau, comprenant l'emploi d'acides aminés.**

(30) Priorité : **19.10.78 FR 7829797**

(43) Date de publication de la demande :
**30.04.80 Bulletin 80/09**

(45) Mention de la délivrance du brevet :
**05.10.83 Bulletin 83/40**

(84) Etats contractants désignés :
**AT BE CH DE GB IT LU NL SE**

(56) Documents cités :
**BE A 458 709
FR A 1 252 400
US A 4 021 538**

**H. JANISTYN : « Handbuch der Kosmetika und Riechstoffe », vol. 1, 2ème édition, 1969, Hüthlg Verlag Heidelberg R.F.A., pages 1117-1119**

**DERWENT JAPANESE PATENTS REPORT, vol. I, no 7, 21 mars 1972, Londres, G.B. 3ème abrégé no 11332 T.**

**Le dossier contient des Informations techniques présentées postérieurement au dépôt de la demande et ne figurant pas dans le présent fascicule.**

(73) Titulaire : **LABORATOIRES SEROBIOLOGIQUES S.A.
F-54 420 Pulnoy (FR)**

(72) Inventeur : **Pauly, Marc
10, rue Joffre
F-57170 Chateau Salins (FR)**

(74) Mandataire : **Weinstein, Zinovi et al
Cabinet Z. WEINSTEIN 20, Avenue de Friedland
F-75008 Paris (FR)**

Jouve, 18, rue St-Denis, 75001 Paris, France

## Composition utilisable notamment comme produit cosmétique permettant un bronzage de la peau, comprenant l'emploi d'acides aminés

La présente invention a essentiellement pour objet une composition utilisable notamment comme produit cosmétique favorisant le bronzage de la peau.

Plus particulièrement, la présente invention concerne une composition, utilisable notamment comme produit cosmétique destinée à être appliquée topiquement sur l'épiderme pour favoriser la photo-pigmentation de celui-ci et/ou sa photo-protection par exposition aux rayonnements solaires ou ultra-violets.

On connaît déjà des compositions favorisant le bronzage de la peau et/ou sa photo-protection. Par exemple, on connaît des préparations dites anti-solaires qui permettent l'exposition au soleil, avec bronzage, tout en protégeant la peau des rayonnements UV B, réputés érythématogènes et dangereux, qui utilisent des substances écran ou filtres solaires préparées par synthèse telles que les salicylates, anthranilates, cinnamates ou esters para-amino benzoïques ou d'origine naturelle obtenues par extraction à partir de végétaux, par exemple huiles végétales, sésame, camomille.

D'autres préparations connues utilisent des photosensibilisants appliqués par voie interne ou topique, préparés par synthèse tels que les furocoumarines ou le 5 ou 8-méthoxypsoralène (M.O.P.), ou d'origine naturelle tels que l'essence de bergamote, la vitamine A ou F.

On connaît également d'autres produits dits bronzants ou tannants sans soleil qui utilisent des agents chimiques tels que dihydroxyacétone, érythrulose, glycérose. De même d'autres préparations connues utilisent des produits colorants et teintés qui procurent un teint hâlé ou bronzé.

Cependant, toutes ces préparations ou compositions connues permettant un bronzage de la peau avec ou sans soleil utilisent des agents étrangers à la peau et présentent des inconvénients très importants et bien connus. En effet, ces agents étrangers sont rarement substantifs. D'autre part, ils sont plus ou moins bien tolérés. En outre, ils doivent être utilisés en applications renouvelées et fréquentes compte tenu de la difficulté de les faire pénétrer dans les couches externes de l'épiderme.

Certaines de ces préparations connues sont même dangereuses, en particulier les préparations à base de photosensibilisants et doivent donc être utilisées avec précautions.

Par ailleurs, on connaît aussi à partir du brevet belge n° 458.709 une composition comportant principalement l'emploi d'une tyrosinase en vue de réaliser un effet bronzant par action oxydante sur la tyrosine présente dans la peau ou s'y trouvant par l'application préalable de substances la contenant. Selon ce document, on utilise principalement la tyrosinase alors que la tyrosine est employée tout à fait accessoirement. En outre, la tyrosinase utilisée selon ce document présente un inconvénient majeur à savoir qu'elle ne peut être utilisée dans les préparations cosmétiques courantes en raison de son instabilité dans sa forme dissoute. Par ailleurs, ce document ne précise pas les conditions d'inhibition de cette enzyme.

Enfin, selon ce document, la tyrosine est employée telle quelle, c'est-à-dire sous une forme libre et en solution aqueuse.

Ce document ne permet pas de résoudre les inconvénients précités car il n'arrive dans le meilleur des cas qu'à fournir une pigmentation superficielle de l'épiderme et la composition selon ce document doit donc être utilisée en application renouvelée et fréquente compte tenu de la difficulté de la faire pénétrer dans les couches externes de l'épiderme.

D'autre part, un ouvrage de Janystin Handbuch der Kosmetika und Riechstoffe, vol. 1/1969, p. 1117-1119 cite un certain nombre d'aminoacides dont la tyrosine qui doit procurer un brunissement de la peau.

L'art antérieur n'a donc pas fourni de solution capable de remédier aux inconvénients précités.

La présente invention a donc pour but de remédier aux inconvénients précités des préparations ou compositions bronzantes connues en fournissant une solution qui permet de réaliser une photo-pigmentation de l'épiderme et/ou sa photo-protection par exposition aux rayonnements solaires ou ultra-violets, qui soit bein tolérée par l'épiderme et évite l'apparition d'érythèmes ou « coups de soleil » douloureux et pouvant avoir des conséquences plus ou moins graves.

En d'autres termes, cette solution selon l'invention doit permettre une diffusion du ou des produits utilisés jusqu'au niveau des mélanocytes simplement par application topique cutanée répétée avec des rayonnements solaires ou UV sur l'épiderme intact tout en évitant une irritation relativement à l'épiderme qui est susceptible de produire des effets secondaires. Cette solution à ce nouveau problème technique doit être de préférence de conception particulièrement simple.

Cette solution consiste selon la présente invention en une composition, utilisable notamment comme produit cosmétique, et dermo-corrective (traitement médical d'achromies de la peau, telles que leucodermies, albinisme partiel, vitiligo) destinée à être appliquée topiquement sur l'épiderme pour favoriser la photo-pigmentation de celui-ci et/ou sa photo-protection par exposition aux rayonnements solaires ou ultra-violets, caractérisée en ce qu'elle comprend un sel complexe de L-tyrosine avec au moins un acide aminé basique choisi parmi l'arginine, l'ornithine, la citrulline, la lysine ou l'hydroxylysine, la concentration en complexe de tyrosine étant comprise entre 0,1 et 10 % en poids de la composition.

Avantageusement, la concentration totale en sel complexe de L-tyrosine est comprise entre environ 0,10 et environ 20 % en poids, de préférence entre environ 1 et environ 10 % en poids de la composition, le reste de la composition étant constitué par un excipient habituel.

Ainsi, la présente invention utilise la L-tyrosine qui joue directement ou indirectement un rôle dans la mélanogenèse. Il en résulte que la composition de la présente invention est parfaitement tolérée par le sujet.

En outre, on doit noter que les propriétés photo-protectrices ou pigmentogènes de la composition de la présente invention ne se développent qu'au contact de l'épiderme lui-même, et qu'après photo-incitation par les rayons solaires ou ultra-violets (soit UV A seule, soit UV A + UV B). Il en résulte que la composition de la présente invention permet de renforcer et d'accélérer le processus naturel de pigmentation, ou mélanogenèse de la peau par exposition aux rayonnements solaires ou UV. Un autre avantage consiste en le fait qu'elle accélère le processus d'auto-photoprotection de la peau contre les rayons ultra-violets réputés dangereux (UV B), cette auto-protection étant liée à l'intensité de la pigmentation et divers facteurs cutanés.

On obtient en outre, de manière inattendue, une photo-protection complémentaire contre les effets dangereux, des expositions solaires brutales ou immodérées en intensité et durée, notamment chez les sujets qui n'y sont pas préparés et permettant de protéger lesdits sujets contre les conséquences des expositions solaires répétées, entraînant le vieillissement précoce de la peau, ou favorisant des cancers cutanés.

La composition de la présente invention permet également d'atténuer les effets douloureux dus aux conséquences d'expositions solaires exagérées, qui provoquent des érythèmes ou « coups de soleil » douloureux et peuvent avoir des conséquences plus ou moins graves pour le sujet.

Un autre avantage très important de la composition de l'invention consiste en ce qu'elle peut être employée pour le traitement médical d'anomalies achromiques de la peau, telles que leucodernies, albinisme partiel, vitiligo en redonnant une pigmentation normale de la peau. La composition de l'invention est en effet particulièrement adaptée à ces applications médicales et elle présente une tolérance et une innocuité parfaites. Elle est facilement utilisée par l'épiderme, même partiellement, pour renforcer l'activité ou l'intensité des processus naturels de photo-pigmentation et d'auto-protection de la peau en réponse aux irradiations solaires ou UV.

Selon une autre caractéristique avantageuse de la présente invention, la composition comprend en outre environ de 0,1 à environ 1 % en poids de tyrosinase.

Suivant encore une autre caractéristique avantageuse de la présente invention, la composition comprend en outre au moins une substance choisie parmi le groupe consistant :

a) des acides aminés courants tels que :

— mono-amines-monocarboxyliques, de préférence : acides amino propioniques $\alpha$ et $\beta$, valine, leucine, isoleucine,
à une concentration comprise entre 0,1 et 10 % en poids de la composition ;

— acides aminés hydroxylés, de préférence :
Sérine, thréonine
à une concentration comprise entre 0,01 et 10 % en poids de la composition ;

— acides mono-aminés dicarboxyliques, de préférence :
acide aspartique à une concentration comprise entre 0,01 et 10 % en poids de la composition,
acide glumatique à une concentration comprise entre 0,01 et 15 % en poids de la composition ;

— acides aminés aromatiques ou cycliques, de préférence : (pourcentage en poids dans la composition)

| | |
|---|---|
| proline | 0,010 à 5 |
| hydroxyproline | 0,01 à 5 |
| histidine | 0,01 à 15 |
| tryptophane | 0,01 à 15 |

— acides aminés à plusieurs fonctions amino-monocarboxyliques, de préférence :
ornithine, citrulline, lysine, hydroxylysine, à une concentration comprise entre 0,1 et 10 % en poids de la composition ;

b) des amino-acides cyto-protecteurs tels que des acides aminés soufrés, de préférence le glutathion, la cystine, la cystéine, ou la méthionine à une concentration comprise entre 0,01 et 15 % en poids de la composition ;

c) des amino-acides photo-protecteurs naturels, ou leur dérivés, de préférence l'acide urocanique et ses sels à une concentration comprise entre 0,1 et 10 % en poids de la composition, de sorte que la concentration totale en sel complexe de L-tyrosine et en amino-acide complémentaire soit comprise entre 0,1 et 20 % en poids de la composition.

On doit noter que l'association ou la combinaison de ces substances homologues précitées, qui sont cutanéo-constitutives de l'épiderme, ont un rôle complémentaire ou synergisant tant pour la photo-pigmentation de l'épiderme que pour sa photo-protection, c'est-à-dire que l'association ou la combinai-

3

0 010 483

son de ces substances permet non seulement de renforcer l'activité de chacune d'elles, mais encore de permettre une meilleure compatibilité avec l'épiderme, de réaliser une meilleure substantivité, solubilité, diffusibilité, pénétrabilité et fixation dans l'épiderme.

Il en résulte qu'un autre avantage de la composition de la présente invention consiste en sa facilité d'emploi, principalement en usage topique (application externe), qu'elle soit solubilisée, dispersée ou diluée dans des excipients courants ou appropriés à ce type d'application, tant en cosmétique, produit d'hygiène que dermatologique, notamment pour le traitement d'anomalies achromiques de la peau (leucodermies, albinisme partiel, vitiligo).

D'autres buts, caractéristiques et avantages de la présente invention apparaîtront plus clairement à la lumière des exemples suivants donnés simplement à titre d'illustration et qui ne sauraient donc en aucune façon limiter la portée de la présente invention.

Dans les exemples, toutes les proportions sont données en pourcentage en poids sauf indications contraires.

Exemple 1

On prépare une composition comprenant comme substance précurseur de mélanine, de la L-tyrosine, sous forme de complexe L-tyrosine-arginine base qui est parfaitement soluble et stable et dont on peut régler le pH sur la neutralité ou sur le pH épidermique en faisant varier les proportions de chacun des constituants.

Par exemple, ce complexe est formé de 47 % de L-tyrosine et 53 % d'arginine base.

Ce complexe est ensuite dilué dans un excipient tel qu'un excipient hydrique, hydroglycolique, hydro-alcoolique, une émulsion huile/eau ou eau/huile, un gel de polymère, un gel organique divers ou tellurique : bentonites, argiles, bentones, hectorites, gels cellulosiques, de façon que ce complexe constitue de 0,1 à 20 % en poids de la composition totale, ce qui permet de préparer une composition utilisable notamment en cosmétique et dans les produits solaires : photo-protecteurs, produits bronzants ayant différentes présentations.

Exemple 2

On prépare une composition ayant les constituants suivants :

— Complexe de :
| | |
|---|---|
| — arginine base | 52,9 |
| — L-tyrosine | 47 |
| — glutathion | 0,1 |

La préparation de cette composition est réalisée de la manière suivante : on mélange tout d'abord la L-tyrosine et l'arginine base de façon à réaliser un complexe L-tyrosine-arginine puis on ajoute le glutathion.

Ensuite, l'association de ces trois composants est diluée dans un excipient habituel du type défini dans l'exemple 1 ci-dessus afin d'obtenir une composition ayant une concentration totale en acides aminés comprise entre 0,1 et 20 % en poids de la composition.

On doit noter que cette association arginine-L-tyrosine glutathion a un rôle photo-protecteur cellulaire particulièrement important. En effet, le glutathion a un rôle protecteur modérateur contre les dégats cellulaires provoqués par les quinones, intermédiaires fortement réactifs, qui interviennent dans le processus de la mélanogénèse, ces quinones étant connues pour affecter gravement l'ARN des cellules épidermiques.

On doit noter qu'on peut substituer le glutathion par d'autres acides aminés soufrés tels que la cystine, la cystéine, la méthionine.

Exemple 3

On prépare une composition ayant les constituants suivants :

— Complexe de :
| | |
|---|---|
| — arginine base | 42 |
| — L-tyrosine | 40 |
| — glutathion | 0,1 |
| — glycine | 17,9 |

Cette composition est préparée en formant d'abord le complexe L-tyrosine-arginine puis en ajoutant le glutathion et la glycine. Ensuite, ce mélange est dilué dans un excipient de façon à ce que la concentration totale en substances soit comprise entre environ 0,1 et 20 % en poids, ledit excipient étant l'un de ceux cités à l'Exemple 1.

4

# 0 010 483

On doit noter que la glycine est un agent synergisant servant de fixateur des dérivés quinoniques et de la DOPA, qui entrent dans le cycle de la mélanogénèse. Cette association renforce donc l'activité protectrice cellulaire (ARN) et de plus, est particulièrement substantive.

## Exemple 4

On prépare une composition ayant les constituants suivants :

— Complexe de :
| | |
|---|---|
| — L-tyrosine | 38 |
| — ornithine | 40 |
| — glutathion | 0,1 |
| — Glycine | 17 |
| — histidine | 4,9 |

La préparation de cette composition est réalisée de manière similaire à celle des exemples précédents de manière à obtenir une concentration totale en substances comprise entre 0,1 et 20 % en poids de la composition.

On doit noter que l'histidine renforce le rôle substantif de la composition ainsi que son rôle photoprotecteur étant donné qu'elle est le précurseur de l'acide urocanique présent au niveau de l'épiderme et des glandes sudoripares.

Ainsi, on peut remplacer l'histidine par l'acide urocanique ou ses dérivés, de préférence l'urocanate d'arginine, l'urocanate d'histidine ou l'urocanate d'arginine-histidine.

On doit noter que l'acide urocanique constitue le photo-protecteur naturel le plus fonctionnel pour la peau en arrêtant partiellement les ultra-violets B qui sont nocifs. D'autre part, l'acide urocanique libre étant peu soluble et peu substantif, ses sels, en particulier ses sels avec des acides aminés, ont l'avantage d'être solubles, actifs sur le plan photo-protecteur et substantifs sur l'épiderme.

## Exemple 5

On prépare une composition donnant d'excellents résultats avec une concentration totale en substances égale à 3 % sous forme de soluté hydro-glycériné ayant les constituants suivants :

| | |
|---|---|
| — complexe L-tyrosine-arginine (47/53) | 0,25 |
| — histidine | 0,03 |
| — urocanate d'arginine | 0,15 |
| — glycine | 2,52 |
| — Glutathion | 0,05 |
| — Glycérine | 20 |
| — eau | QSP 100 |

## Exemple 6

On prépare une composition comprenant les constituants suivants : (substances totales : 3 % en poids)

— Complexe de :
| | |
|---|---|
| — L-tyrosine | 0,12 |
| — L-arginine | 0,12 |
| — glutathion | 0,01 |
| — acide urocanique | 0,15 |
| — L-arginine | 0,05 |
| — tyrosinase | 0,1 |
| — histidine | 0,03 |
| — glycine | 2,42 |
| — excipient émulsionné | 97 |

## Exemple 7

On prépare une composition ayant une concentration totale en substances égale à 9 % avec les constituants suivants :

— Complexe de :
| | |
|---|---|
| — L-tyrosine | 1,00 |
| — L-arginine | 0,50 |

5

| | |
|---|---|
| — ornithine ou citrulline ou lysine | 0,50 |
| — acide urocanique | 1,00 |
| — L-arginine | 0,50 |
| — Lysine | 0,40 |
| — histidine | 2,10 |
| — glycine | 3,00 |
| — excipient émulsionné | QSP 100 |

Exemple 8

On prépare une composition formant une préparation protectrice solaire ayant une concentration totale en substances égale à 3 % de la composition, comprenant les constituants suivants :

| | |
|---|---|
| — complexe L-tyrosine-arginine | 0,25 |
| — urocanate d'arginine | 0,20 |
| — histidine | 0,03 |
| — glycine | 2,51 |
| — glutathion | 0,01 |
| — excipient | 97 |

L'excipient est constitué par les composés suivants :

| | |
|---|---|
| — glycéryl monostéarate | 14 |
| — dérivé lanoline | 5 |
| — diéthyl sébaçate | 10 |
| — isopropyl myristate | 15 |
| — dioxyde de titane | 2 |
| — glycérine | 6 |
| — eau | 45 |

Ainsi que des quantités mineures d'agents de préservation.

On doit noter que dans les formulations précitées données simplement à titre d'exemple, l'excipient ne joue qu'un rôle de véhicule, cosmétiquement ou dermatologiquement adapté, sans influence notable sur l'activité des complexes ou associations des substances homologues faisant l'objet de la présente invention.

Dans certains cas particuliers, on peut renforcer la photo-protection en ajoutant des filtres solaires de synthèse tels que des cinnamates en quantités comprises entre 0,10 et 5 % en poids de la composition ou encore on peut ajouter des renforçateurs de tannage sans soleil tels que D.H.A. (Dihydroxyacétone) à une concentration comprise entre 0,3 et 5 % en poids de la composition.

Pour démontrer les actions physiologiques de la composition de la présente invention, des essais cliniques ont été réalisés sur des patients souffrant d'achromies de la peau. Les patients traités souffraient de vitiligo.

Pour traiter ces patients, on a préparé une préparation topique de la composition selon la présente invention contenant de la tyrosine, comprenant un soluté hydroglycolique à 20 % de propyleneglycol dans lequel on a dissous à une teneur de 3 % en poids la combinaison suivante de substances :

| | |
|---|---|
| — Complexe de : | |
| — arginine base | 4,5 |
| — L-tyrosine | 4,0 |
| — acide urocanique | 5,0 |
| — histidine HCl | 0,1 |
| — glutathion | 0,02 |
| — acide glutamique | 86,38 |
| TOTAL | 100,00 |

Pour le traitement du vitiligo, des applications toniques de la composition ci-dessus ont été réalisées toutes les 24 heures sur les zones apigmentées ou irrégulièrement pigmentées, et on a réalisé ensuite des irradiations pendant une période de temps d'environ 10 minutes — 30 minutes avec une lampe UVR de 250 watts. L'émission de rayons UV comprend environ : 20 % de rayons UVB et 80 % de rayons UVA.

Cinq patients souffrant de vitiligo ont été traités avec la composition pharmaceutique ci-dessus en réalisant six irradiations successives de 20 minutes, chacune espacée de 24 heures, avec la lampe UVR ci-dessus.

Les résultats ont été tout à fait surprenants dès la première irradiation, par repigmentation marginale des zones apigmentées.

Après la seconde irradiation, la repigmentation apparaît sur la totalité de la superficie des zones dépigmentées traitées.

Pendant les irradiations restantes, la repigmentation s'accroît en intensité, sans apparition d'érythèmes.

Ces résultats sont particulièrement inattendus puisqu'il a été jusqu'à présent très difficile de trouver un traitement efficace du vitiligo. La composition selon la présente invention par applications topiques permet de résoudre un tel problème avec une simple irradiation UV pendant une période de temps courte ce qui met clairement en évidence l'activité photo-pigmentogène de la composition selon la présente invention avec exposition UVA-UVB.

D'autre part, des essais de toxicité ont été réalisés sur des souris en étudiant l'irritation primaire cutanée selon la méthode officielle d'analyse des produits cosmétiques et des produits de beauté publiée dans le Journal Officiel français du 21 Avril 1971. Egalement, une étude a été réalisée en ce qui concerne l'indice d'irritation occulaire primaire sur des souris avec l'indice d'aggressivité cutanée superficielle de la composition selon la présente invention par des applications itératives.

Pour les trois études ci-dessus, réalisées selon les procédures officielles avec la même composition que celle employée pour le traitement précité du vitiligo, on n'a pas observé d'effets irritants de sorte qu'on peut en conclure que la composition de la présente invention n'a aucun effet toxique.

**Revendications** (pour les Etats Contractants : BE, CH, DE, GB, IL, LU, NL, SE)

1. Composition utilisable comme produit cosmétique et dermo-corrective (traitement médical d'achromie de la peau, telles que leucodermies, albinisme partiel, vitiligo), destinée à être appliquée topiquement sur l'épiderme pour favoriser la photo-pigmentation de celui-ci et/ou sa photo-protection par exposition aux rayonnements solaires ou ultra-violets, caractérisée en ce qu'elle comprend un sel complexe de L-tyrosine avec au moins un acide aminé basique choisi parmi l'arginine, l'ornithine, la citrulline, la lysine, ou l'hydroxylysine, la concentration en complexe de tyrosine étant comprise entre 0,1 et 10 % en poids de la composition.

2. Composition selon la revendication 1, caractérisée en ce qu'elle comprend en outre au moins un amino-acide choisi parmi le groupe des acides aminés courants comprenant des acides aminés hydroxylés, des acides mono-aminés monocarboxyliques, des acides monoaminés dicarboxyliques, des acides aminés aromatiques ou cycliques, des acides aminés à plusieurs fonctions amino, monocarboxyliques ; des amino-acides cytoprotecteurs, des amino-acides photo-protecteurs naturels ou leurs dérivés, la concentration totale en complexe de L-tyrosine et en amino-acide complémentaire étant comprise entre environ 0,10 et 20 % en poids, de préférence entre environ 1 et environ 10 % en poids de la composition, le reste étant constitué par un excipient habituel.

3. Composition selon la revendication 2, caractérisée en ce que les acides monoaminés monocarxyliques sont choisis parmi les acides amino-propioniques $\alpha$ et $\beta$, la valine, leucine, isoleucine, à une concentration comprise entre 0,1 et 10 % en poids de la composition ; les acides aminés hydroxylés sont choisis parmi la sérine, thréonine à une concentration comprise entre 0,01 et 10 % en poids de la composition ; les acides monoaminés dicarboxyliques sont choisis parmi l'acide aspartique (0,01 à 10 % en poids de la composition), l'acide glutamique (0,01 à 15 % en poids de la composition) ; les acides aminés aromatiques ou cycliques sont choisis parmi la proline (0,10 à 5 % en poids), hydroxyproline (0,01 à 5 % en poids), l'histidine (0,01 à 15 % en poids), le tryptophane (0,01 à 15 % en poids) les acides aminés à plusieurs fonctions amino, monocarboxyliques sont choisis parmi l'ornithine, la citrulline, la lysine, l'hydroxylysine à une concentration comprise entre 0,01 et 10 % en poids de la composition ; les amino-acides cyto-protecteurs sont choisis parmi le glutathion, la cystine, la cystéine, la méthionine à une concentration comprise entre 0,01 et 15 % en poids de la composition ; les amino-acides photo-protecteurs naturels ou leurs dérivés sont choisis parmi l'acide urocanique et ses sels à une concentration comprise entre 0,1 et 10 % en poids de la composition.

4. Composition selon la revendication 3, caractérisée en ce que l'acide urocanique est utilisé sous forme de dérivé complexe avec un ou plusieurs acides aminés basiques, de préférence choisi parmi l'histidine, l'arginine ou les deux.

5. Composition selon l'une des revendications 1 à 4, caractérisée en ce qu'elle comprend en outre environ de 0,1 à environ 1 % en poids de tyrosinase.

6. Composition selon l'une des revendications 1 à 5, caractérisée en ce que l'acide aminé basique combiné avec la L-tyrosine est l'arginine.

7. Composition selon l'une des revendications 1 à 6, caractérisée en ce qu'elle comprend (pourcentage en poids) :

— Complexe de :
  — L-tyrosine      40
  — arginine base      42
  — glutathion      0,1
  — glycine      17,9

ces constituants en mélange étant dilués dans un excipient choisi parmi un excipient hydrique, hydroglycolique, hydroalcoolique, une émulsion huile/eau ou eau/huile, un gel de polymère, un gel organique divers ou tellurique : bentonites, argiles, bentones, hectorites, gels cellulosiques.

8. Composition selon l'une des revendications 1 à 6, caractérisée en ce qu'elle a la composition suivante en pourcentage en poids :

| | |
|---|---:|
| — complexe L-tyrosine-arginine | 0,25 |
| — urocanate d'arginine | 0,20 |
| — histidine | 0,03 |
| — glycine | 2,51 |
| — glutathion | 0,01 |
| — excipient | 97 |

l'excipient étant constitué par les composants suivants :

| | |
|---|---:|
| — glycéryl monostéarate | 14 |
| — dérivé de lanoline | 5 |
| — diéthyl sébaçate | 10 |
| — isopropyl myristate | 15 |
| — dioxyde de titane | 2 |
| — glycérine | 6 |
| — eau | 45 |

9. Composition selon l'une des revendications 1 à 6, caractérisée en ce qu'elle comprend un soluté hydroglycolique à 20 % de propylèneglycol contenant à une teneur de 3 % en poids la combinaison suivante :

| | |
|---|---:|
| — Complexe de : | |
| — arginine base | 4,5 |
| — L-tyrosine | 4,0 |
| — acide urocanique | 5,0 |
| — histidine HCl | 0,1 |
| — glutathion | 0,02 |
| — acide glutamique | 86,38 |

**Revendications** (pour l'Etat Contractant AT)

1. Procédé de préparation d'une composition utilisable comme produit cosmétique, et dermo-corrective, caractérisé en ce qu'il comprend l'incorporation dans ladite composition d'un sel complexe de L-tyrosine avec au moins un acide aminé basique choisi parmi l'arginine, l'ornithine, la citrulline, la lysine, ou l'hydroxylysine de sorte que la concentration en complexe de tyrosine soit comprise entre 0,1 et 10 % en poids de la composition.

2. Procédé selon la revendication 1, caractérisé en ce qu'on introduit en outre au moins un amino acide choisi parmi le groupe des acides aminés courants comprenant des acides aminés hydroxylés, des acides mono-aminés monocarboxyliques, des acides monoaminés dicarboxyliques, des acides aminés aromatiques ou cycliques, des acides aminés à plusieurs fonctions amino, monocarboxyliques ; des amino-acides cyto-protecteurs ; des amino acides photoprotecteurs naturels ou leurs dérivés, de sorte que la concentration totale en complexe de tyrosine et en amino-acide complémentaire soit comprise entre environ 0,10 et 20 % en poids, de préférence entre environ 1 et environ 10 % en poids de la composition, le reste étant constitué par un excipient habituel.

3. Procédé selon la revendication 2, caractérisé en ce que les acides monoaminés monocarboxyliques sont choisis parmi les acides amino-propioniques $\alpha$ et $\beta$, la valine, leucine, isoleucine, à une concentration comprise entre 0,1 et 10 % en poids de la composition ; les acides aminés hydroxylés sont choisis parmi la sérine, thréonine à une concentration comprise entre 0,01 et 10 % en poids de la composition ; les acides monoaminés dicarboxyliques sont choisis parmi l'acide aspartique (0,01 à 10 % en poids de la composition), l'acide glutamique (0,01 à 15 % en poids de la composition); les acides aminés aromatiques ou cycliques sont choisis parmi la proline (0,10 à 5 % en poids), hydroxyproline (0,01 à 5 % en poids), histidine (0,01 à 15 % en poids), le tryptophane (0,01 à 15 % en poids), les acides aminés à plusieurs fonctions amino, monocarboxyliques sont choisis parmi l'ornithine, citrulline, lysine, hydroxylysine à une concentration comprise entre 0,01 et 10 % en poids de la composition ; les amino-acides cyto-protecteurs sont choisis parmi le glutathion, cystine, cystéine, méthionine à une concentration comprise entre 0,01 et 15 % en poids de la composition ; les amino-acides photo-protecteurs naturels ou leurs dérivés sont choisis parmi l'acide urocanique et ses sels à une concentration comprise entre 0,1 et 10 % en

8

poids de la composition.

4. Procédé selon la revendication 3, caractérisé en ce que l'acide urocanique est utilisé sous forme de dérivé complexe avec un ou plusieurs acides aminés basiques, de préférence choisi parmi l'histidine, l'arginine ou les deux.

5. Procédé selon l'une des revendications 1 à 4, caractérisé en ce qu'on introduit dans la composition en outre environ 0,1 à environ 1 % en poids de tyrosinase.

6. Procédé selon l'une des revendications 1 à 5, caractérisé en ce que l'on introduit un complexe de L-tyrosine et d'arginine.

7. Procédé selon l'une des revendications 1 à 6, caractérisé en ce que l'on introduit dans la composition les composants suivants (pourcentages en poids) :

| | |
|---|---|
| — Complexe de : | |
|   — L-tyrosine | 40 |
|   — arginine base | 42 |
| — glutathion | 0,1 |
| — glycine | 17,9 |

et en ce qu'on dilue ces constituants en mélange dans un excipient choisi parmi un excipient hydrique, hydroglycolique, hydroalcoolique, une émulsion huile/eau ou eau/huile, un gel de polymère, un gel organique divers ou tellurique : bentonites, argiles, bentones, hectorites, gels cellulosiques.

8. Procédé selon l'une des revendications 1 à 7, caractérisé en ce que l'on introduit dans la composition les composants suivants en pourcentage en poids :

| | |
|---|---|
| — complexe L-tyrosine-arginine | 0,25 |
| — urocanate d'arginine | 0,20 |
| — histidine | 0,03 |
| — glycine | 2,51 |
| — glutathion | 0,01 |
| — excipient | 97 |

l'excipient étant constitué par les composants suivants :

| | |
|---|---|
| — glycéryl monostéarate | 14 |
| — dérivé de lanoline | 5 |
| — diéthyl sébaçate | 10 |
| — isopropyl myristate | 15 |
| — dioxyde de titane | 2 |
| — glycérine | 6 |
| — eau | 45 |

9. Procédé selon l'une des revendications 1 à 8, caractérisé en ce qu'on prépare un soluté hydroglycolique à 20 % de propylèneglycol contenant à une teneur de 3 % en poids la combinaison suivantes :

| | |
|---|---|
| — Complexe de : | |
|   — arginine base | 4,5 |
|   — L-tyrosine | 4,0 |
| — acide urocanique | 5,0 |
| — histidine HCl | 0,1 |
| — glutathion | 0,02 |
| — acide glutamique | 86,38 |

**Claims** (for the Contracting States : BE, CH, DE, GB, IL, LU, NL, SE)

1. A composition usable as a cosmetic product, and as a dermo-corrective (medical treatment of skin achromy, such as leucodermia, partial albinism, vitiligo), intended to be applied topically to the epidermis to favour the photo-pigmentation of the latter and/or its photo-protection by exposure to sun or ultraviolet rays, characterized in that it comprises a complex salt of L-tyrosine with at least one basic aminoacid chosen among arginine, ornithine, citrulline, lysine, or hydroxylysine, the tyrosine complex concentration being comprised between 0.1 and 10 % by weight of the composition.

2. A composition according to claim 1, characterized in that it also comprises at least one amino-acid chosen from the group of amino-acids in current use comprising hydroxylated amino-acids, monocarboxy-

9

# 0 010 483

lic monoamino-acids, dicarboxylic monoamino-acids, aromatic or cyclic amino-acids, amino-acids with several amino-monocarboxylic functions ; cyto-protective amino-acids, natural photo-protective amino-acids or derivatives thereof, the total concentration of tyrosine complex and of complementary amino-acid being comprised between about 0.10 and 20 % by weight, preferably between about 1 and about 10 % by weight of the composition, the balance being constituted by a usual excipient.

3. A composition according to claim 2, characterized in that the monocarboxylic monoamino-acids are chosen among the $\alpha$ and $\beta$ aminopropionic acids, valine, leucine, isoleucine, at a concentration comprised between 0.1 and 10 % by weight of the composition ; the hydroxylated amino-acids are chosen among serine, threonine at a concentration comprised between 0.01 and 10 % by weight of the composition ; the dicarboxylic monoamino-acids are chosen among aspartic acid (0.01 to 10 % by weight of the composition), glutamic acid (0.01 to 15 % by weight of the composition) ; the aromatic or cyclic amino-acids are chosen among proline (0.10 to 5 % by weight), hydroxyproline (0.01 to 5 % by weight), histidine (0.01 to 15 % by weight), tryptophane (0.01 to 15 % by weight) ; the amino-acids with several amino-monocarboxylic functions are chosen among ornithine, citrulline, lysine, hydroxylysine at a concentration comprised between 0.01 and 10 % by weight of the composition ; the cyto-protective amino-acids are chosen among glutathion, cystine, cysteine, methionine at a concentration comprised between 0.01 and 15 % by weight of the composition ; the natural photo-protective amino-acids or their derivatives are chosen among urocanic acid and the salts there of at a concentration comprised between 0.1 and 10 % by weight of the composition.

4. A composition according to claim 3, characterized in that the urocanic acid is used in the form of a complex derivative with one or several basic aminoacids, preferably chosen among histidine, arginine or both.

5. A composition according to one of claims 1 to 4, characterized in that it also comprises about 0.1 to about 1 % by weight of tyrosinase.

6. A composition according to one of claims 1 to 5, characterized in that the basic amino-acid combined with L-tyrosine is arginine.

7. A composition according to one of claims 1 to 6, characterized in that it comprises (weight percent) ;

— complex of :
| | |
|---|---|
| — L-tyrosine | 40 |
| — basic arginine | 42 |
| — glutathion | 0.1 |
| — glycine | 17.9 |

these constituents in the mixed state being diluted with an excipient chosen among a hydrous, hydroglycolic, hydroalcoholic excipient, an oil/water or water/oil emulsion, a polymer gel, any organic or telluric gel : bentonites, clays, bentones, hectorites, cellulose gels.

8. A composition according to one of claims 1 to 6, characterized in that it has a following composition (weight percent) :

| | |
|---|---|
| — L-tyrosine-arginine complex | 0.25 |
| — arginine urocanate | 0.20 |
| — histidine | 0.03 |
| — glycine | 2.51 |
| — glutathion | 0.01 |
| — excipient | 97 |

the excipient being constituted by the following components :

| | |
|---|---|
| — glyceryl monostearate | 14 |
| — lanoline derivative | 5 |
| — diethyl sebacate | 10 |
| — isopropylmyristate | 15 |
| — titane dioxyde | 2 |
| — glycerine | 6 |
| — water | 45 |

9. A composition according to one of claims 1 to 6, characterized in that it comprises a 20 % propyleneglycol hydroglycolic solute containing at a content of 3 % by weight the following combination :

— complex of :

10

| | |
|---|---:|
| — basic arginine | 4.5 |
| — L-tyrosine | 4.0 |
| — urocanic acid | 5.0 |
| — histidine HCl | 0.1 |
| — glutathion | 0.02 |
| — glutamic acid | 86.38 |

**Claims** (for the Contracting State AT)

1. A method for producing a composition usable as a cosmetic product, and as a dermo-corrective, characterized in that it comprises the incorporation into the said composition of a complex salt of L-tyrosine with at least one basic amino-acid chosen among arginine, ornithine, citrulline, lysine, or hydroxylysine in such a manner that the concentration of the tyrosine complex be comprised between 0.1 and 10 % by weight of the composition.

2. A method according to claim 1, characterized in that there is also introduced at least one amino-acid chosen from the group of amino-acids in current use comprising hydroxylated amino-acids, monocarboxylic monoamino-acids, dicarboxylic monoamino-acids, aromatic or cyclic amino-acids, amino-acids with several amino-monocarboxylic functions ; cyto-protective amino-acids, natural photo-protective amino-acids or derivatives thereof, in such a manner that the total concentration of tyrosine complex and of complementary amino-acid be comprised between about 0.10 and 20 % by weight, preferably between about 1 and about 10 % by weight of the composition, the balance being constituted by a usual excipient.

3. A method according to claim 2, characterized in that the monocarboxylic monoamino-acids are chosen among the α and β aminopropionic acids, valine, leucine, isoleucine, at a concentration comprised between 0.1 and 10 % by weight of the composition ; the hydroxylated amino-acids are chosen among serine, threonine, at a concentration comprised between 0.01 and 10 % by weight of the composition ; the dicarboxylic monoamino-acids are chosen among aspartic acid (0.01 to 10 % by weight of the composition), glutamic acid (0.01 to 15 % by weight of the composition), the aromatic or cyclic amino-acids are chosen among proline (0.10 to 5 % by weight), hydroxyproline (0.01 to 5 % by weight), histidine (0.01 to 15 % by weight), tryptophane (0.01 to 15 % by weight) ; the amino-acids with several amino-monocarboxylic functions are chosen among ornithine, citrulline, lysine, hydroxylysine at a concentration comprised between 0.01 and 10 % by weight of the composition ; the cyto-protective amino-acids are chosen among glutathion, cystine, cysteine, methionine at a concentration comprised between 0.01 and 15 % by weight of the composition ; the natural photo-protective amino-acids or their derivatives are chosen among urocanic acid and the salts thereof at a concentration comprised between 0.1 and 10 % by weight of the composition.

4. A method according to claim 3, characterized in that the urocanic acid is used in the form of a complex derivative with one or several basic aminoacids, preferably chosen among histidine, arginine, or both.

5. A method according to one of claims 1 to 4, characterized in that there is also introduced into the composition about 0.1 to about 1 % by weight of tyrosinase.

6. A method according to one of claims 1 to 5, characterized in that there is introduced a complex of L-tyrosine and arginine.

7. A method according to one of claims 1 to 6, characterized in that there are introduced into the composition the following components (weight percent) :

| | |
|---|---:|
| — complex of : | |
| — L-tyrosine | 40 |
| — basic arginine | 42 |
| — glutathion | 0.1 |
| — glycine | 17.9 |

and in that these constituents in the mixed state are diluted with an excipient chosen among a hydrous, hydroglycolic, hydroalcoholic excipient, an oil/water or water/oil emulsion, a polymer gel, any organic or telluric gel : bentonites, clays, bentones, hectorites, cellulose gels.

8. A method according to one of claims 1 to 7, characterized in that there are introduced into the composition the following components (weight percent) :

| | |
|---|---:|
| — L-tyrosine-arginine complex | 0.25 |
| — arginine urocanate | 0.20 |
| — histidine | 0.03 |
| — glycine | 2.51 |
| — glutathion | 0.01 |
| — excipient | 97 |

**0 010 483**

the excipient being constituted by the following components :

| | |
|---|---|
| — glyceryl monostearate | 14 |
| — lanoline derivative | 5 |
| — diethyl sebacate | 10 |
| — isopropylmyristate | 15 |
| — titane dioxyde | 2 |
| — glycerine | 6 |
| — water | 45 |

9. A method according to one of claims 1 to 8, characterized in that there is produced a 20 % propyleneglycol hydroglycolic solute containing at a content of 3 % by weight the following combination :

| | |
|---|---|
| — complex of : | |
| — basic arginine | 4.5 |
| — L-tyrosine | 4.0 |
| — urocanic acid | 5.0 |
| — histidine HCl | 0.1 |
| — glutathion | 0.02 |
| — glutamic acid | 86.38 |

**Ansprüche** (für die Vertragsstaaten : BE, CH, DE, GB, IL, LU, NL, SE)

1. Hautverbessernde, als kosmetisches Mittel einsetzbare Zusammensetzung (zur ärztlichen Behandlung von Achromie der Haut wie Leukoderma, Teilalbinismus, Vitiligo), welche bestimmt ist, örtlich auf die Oberhaut aufgetragen zu werden, um die Photopigmentierung derselben und/oder deren Schutz gegen Lichteinwirkungen durch Sonnenlicht- bzw. Ultraviolettbestrahlung zu fördern, dadurch gekennzeichnet, dass sie ein Komplexsalz von L-Tyrosin mit wenigstens einer basischen Aminosäure umfasst, die unter Arginin, Ornithin, Zitrullin, Lysin oder Hydroxylysin gewählt wird, wobei die Konzentration an Tyrosinkomplex gewichtsmässig zwischen 0,1 und 10 % der Zusammensetzung liegt.

2. Zusammensetzung nach Anspruch 1, dadurch gekennzeichnet, dass sie ausserdem wenigstens eine Aminosäure umfasst, die unter der Gruppe der gebräuchlichen Aminosäuren bestehend aus Hydroxy-Aminosäuren, Monoamino-Monokarbonsäuren, Monoamino-Dikarbonsäuren, aromatischen oder zyklischen Aminosäuren, Aminosäuren mit mehreren Amino-, Monokarbonfunktionen ; zellschützende Aminosäuren, natürliche lichtschützende Aminosäuren oder deren Derivaten gewählt wird, wobei die Gesamtkonzentration an L-Tyrosinkomplex und an Komplement-Aminosäure gewichtsmässig zwischen ungefähr 0,10 und 20 % und vorzugsweise gewichtsmässig zwischen ungefähr 1 und ungefähr 10 % der Zusammensetzung liegt, wobei der Rest aus einem gewöhnlichen Auflösungsmittel besteht.

3. Zusammensetzung nach Anspruch 2, dadurch gekennzeichnet, dass die Monoamino-Monokarbonsäuren unter den α- und β-Aminopropionsäuren, Valin, Leucin, Isoleucin, mit einer gewichtsmässigen Konzentration die zwischen 0,1 und 10 % der Zusammensetzung liegt, gewählt werden ; die Hydroxy-Aminosäuren unter Serin, Threonin, mit einer gewichtsmässigen Konzentration die zwischen 0,01 und 10 % der Zusammensetzung liegt, gewählt werden ; die Monoamino-Dikarbonsäuren unter der Asparaginsäure (0,01 bis 10 Gew.-% der Zusammensetzung), der Glutaminsäure (0,01 bis 15 Gew.-% der Zusammensetzung) gewählt werden ; die aromatischen bzw. zyklischen Aminosäuren unter Prolin (0,10 bis 5 Gew.-%), Hydroxyprolin (0,01 bis 5 Gew.-%), Histidin (0,01 bis 15 Gew.-%), Tryptophan (0,01 bis 15 Gew.-%) gewählt werden ; die Aminosäuren mit mehreren Amino-, Monokarbonfunktionen unter Ornithin, Zitrullin, Lysin, Hydroxylysin mit einer gewichtsmässigen Konzentration, die zwischen 0,01 und 10 % der Zusammensetzung liegt, gewählt werden ; die zellschützenden Aminosäuren unter Glutathion, Zystin, Zystein, Methionin, mit einer gewichtsmässigen Konzentration, die zwischen 0,01 und 15 % der Zusammensetzung liegt, gewählt werden ; die natürlichen lichtschützenden Aminosäuren oder deren Derivate unter der urokanischen Säure und ihren Sälzen, mit einer gewichtsmässigen Konzentration, die zwischen 0,1 und 10 % der Zusammensetzung liegt, gewählt werden.

4. Zusammensetzung nach Anspruch 3, dadurch gekennzeichnet, dass die urokanische Säure in Form eines komplexen Derivates mit einer oder mehreren basischen Aminosäuren, die vorzugsweise unter Histidin, Arginin oder beiden gewählt wird bzw. werden, verwendet wird.

5. Zusammensetzung nach einem der Ansprüche 1 bis 4, dadurch gekennzeichnet, dass sie ausserdem ungefähr 0,1 bis ungefähr 1 Gew.-% an Tyrosinase enthält.

6. Zusammensetzung nach einem der Ansprüche 1 bis 5, dadurch gekennzeichnet, dass die mit L-Tyrosin verbundene basische Aminosäure Arginin ist.

7. Zusammensetzung nach einem der Ansprüche 1 bis 6, dadurch gekennzeichnet, dass sie (im Gewichtsprozentsatz) :

12

— Komplex von :
— L-Tyrosin     40
— basischem Arginin     42
— Gluthation     0,1
— Glyzin     17,9

umfasst, wobei diese Bestandteile im Gemisch in einem Auflösungsmittel verdünnt werden, welches unter einem wässrigen, hydroglykolischen, hydroalkoholischen Auflösungsmittel, einer Öl-in-Wasser oder Wasser-in-Öl Emulsion, einem Polymergel, einem verschiedenartig organischen oder Tellurgel : Bentonite, Tone, Bentone, Hektorite, Gele aus Zellulose, gewählt wird.

8. Zusammensetzung nach einem der Ansprüche 1 bis 6, dadurch gekennzeichnet, dass sie die folgende gewichtsprozentsatzmässige Zusammensetzung hat :

— Komplex von L-Tyrosin-Arginin     0,25
— Argininurokanat     0,20
— Histidin     0,03
— Glyzin     2,51
— Glutathion     0,01
— Auflösungsmittel     97

wobei das Auflösungsmittel aus folgenden Bestandteilen besteht :

— Glyzeryl-Monostearat     14
— Lanolinderivat     5
— Diethyl-Sebazat     10
— Isopropylmyristat     15
— Titan-Dioxyd     2
— Glyzerin     6
— Wasser     45

9. Zusammensetzung nach einem der Ansprüche 1 bis 6, dadurch gekennzeichnet, dass sie ein hydroglykolisches Gelöstes mit 20 % Propylenglykol umfasst, welches bei einem Gehalt von 3 Gew.-% die folgende Verbindung gewichtsprozentsatzmässig enthält :

— Komplex :
— von basischem Arginin     4,5
— und von L-Tyrosin     4,0
— urokanische Säure     5,0
— HCl-Histidin     0,1
— Glutathion     0,02
— Glutaminsäure     86,38

**Ansprüche** (für den Vertragsstaat AT)

1. Verfahren zur Herstellung einer als kosmetisches Erzeugnis verwendbaren, hautverbessernden Zusammensetzung, dadurch gekennzeichnet, dass es darin besteht, dieser Zusammensetzung ein Komplexsalz von L-Tyrosin mit wenigstens einer unter Arginin, Ornithin, Zitrullin, Lysin oder Hydroxylysin gewählten basichen Aminosäure zuzusetzen, so dass die Konzentration an Tyrosinkomplex zwischen 0,1 und 10 Gew.-% der Zusammensetzung liegt.

2. Verfahren nach Anspruch 1, dadurch gekennzeichnet, dass man ausserdem wenigstens eine Aminosäure zusetzt, die unter der Gruppe der gebräuchlichen Aminosäuren bestehend aus Hydroxy-Aminosäuren, Monoamino-Monokarbonsäuren, Monoamino-Dikarbonsäuren, aromatischen oder zyklischen Aminosäuren, Aminosäuren mit mehreren Amino-, Monokarbonfunktionen ; zellschützende Aminosäuren, natürliche lichtschützende Aminosäuren oder deren Derivaten gewählt wird, wobei die Gesamtkonzentration an L-Tyrosinkomplex und an Komplement-Aminosäure zwischen ungefähr 0,10 und 20 Gew.-% und vorzugsweise zwischen ungefähr 1 und ungefähr 10 Gew.-% der Zusammensetzung liegt, wobei der Rest aus einem gewöhnlichen Auflösungsmittel besteht.

3. Verfahren nach Anspruch 2, dadurch gekennzeichnet, dass die Monoamino-Monokarbonsäuren unter den $\alpha$- und $\beta$-Aminopropionsäuren, Valin, Leucin, Isoleucin mit einer Konzentration, die zwischen 0,1 und 10 Gew.-% der Zusammensetzung liegt, gewählt werden ; die Hydroxy-Aminosäuren unter Serin, Threonin mit einer Konzentration die zwischen 0,01 und 10 Gew.-% der Zusammensetzung liegt, gewählt werden ; die Monoamino-Dikarbonsäuren unter der Asparaginsäure (0,01 bis 10 Gew.-% der Zusammensetzung), der Glutaminsäure (0,01 bis 15 Gew.-% der Zusammensetzung) gewählt werden ; die aromatischen bzw. zyklischen Aminosäuren unter Prolin (0,10 bis 5 Gew.-%), Hydroxyprolin (0,01 bis 5 Gew.-%), Histidin (0,01 bis 15 Gew.-%), Tryptophan (0,01 bis 15 Gew.-%) gewählt werden ; die Aminosäuren mit mehreren Amino-, Monokarbonfunktionen unter Ornithin, Zitrullin, Lysin, Hydroxylysin mit einer Konzentration, die zwischen 0,01 und 10 Gew.-% der Zusammensetzung liegt, gewählt werden ; die zellschützenden Aminosäuren unter Glutathion, Zystin, Zystein, Methionin, mit einer Konzentration, die zwischen 0,01 und 15 Gew.-% der Zusammensetzung liegt, gewählt werden ; die natürlichen lichtschützenden Aminosäuren oder deren Derivate unter der urokanischen Säure und ihren Sälzen mit einer Konzentration, die zwischen 0,1 und 10 Gew.-% der Zusammensetzung liegt, gewählt werden.

4. Verfahren nach Anspruch 3, dadurch gekennzeichnet, dass die urokanische Säure in Form eines komplexen Derivates mit einer oder mehreren basischen Aminosäuren, die vorzugweise unter Histidin, Arginin oder beiden gewählt wird bzw. werden, verwendet wird.

5. Verfahren nach einem der Ansprüche 1 bis 4, dadurch gekennzeichnet, dass man der Zusammensetzung ausserdem ungefähr 0,1 bis ungefähr 1 Gew.-% Tyrosinase zusetzt.

6. Verfahren nach einem der Ansprüche 1 bis 5, dadurch gekennzeichnet, dass man ein Komplex von L-Tyrosin und von Arginin zusetzt.

7. Verfahren nach einem der Ansprüche 1 bis 6, dadurch gekennzeichnet, dass man der Zusammensetzung (gewichtsprozentsatzmässig) folgende Bestandteile zusetzt :

| | |
|---|---|
| — Komplex von : | |
| — L-Tyrosin | 40 |
| — basischem Arginin | 42 |
| — Glutathion | 0,1 |
| — Glyzin | 17,9 |

und dass man diese Bestandteile im Gemisch in einem Auflösungsmittel verdünnt, welches unter einem wässrigen, hydroglykolischen, hydroalkoholischen Auflösungsmittel, einer Öl-in-Wasser oder Wasser-in-Öl Emulsion, einem Polymergel, einem verschiedenartig organischen oder Tellurgel : Bentonite, Tone, Bentone, Hektorite, Gele aus Zellulose, gewählt wird.

8. Verfahren nach einem der Ansprüche 1 bis 7, dadurch gekennzeichnet, dass man der Zusammensetzung (gewichtsprozentsatzmässig) folgende Bestandteile zusetzt :

| | |
|---|---|
| — Komplex von L-Tyrosin-Arginin | 0,25 |
| — Argininurokanat | 0,20 |
| — Histidin | 0,03 |
| — Glyzin | 2,51 |
| — Glutathion | 0,01 |
| — Auflösungsmittel | 97 |

wobei das Auflösungsmittel aus folgenden Bestandteilen besteht :

| | |
|---|---|
| — Glyzeryl-Monostearat | 14 |
| — Lanolinderivat | 5 |
| — Diethyl-Sebazat | 10 |
| — Isopropyl-Myristat | 15 |
| — Titan-Dioxyd | 2 |
| — Glyzerin | 6 |
| — Wasser | 45 |

9. Verfahren nach einem der Ansprüche 1 bis 8, dadurch gekennzeichnet, dass man ein hydroglykolisches Gelöstes mit 20 % Propylenglykol vorbereitet, welches bei einem Gehalt von 3 Gew.-%, die folgende Verbindung enthält :

| | |
|---|---|
| — Komplex : | |
| — von basischem Arginin | 4,5 |
| — und von L-Tyrosin | 4,0 |
| — urokanische Säure | 5,0 |
| — HCL-Histidin | 0,1 |
| — Glutathion | 0,02 |
| — Glutaminsäure | 86,38 |

14